# EUROPEAN PATENT APPLICATION

(11) **EP 3 342 373 A1**
(43) Date of publication of application: **04.07.2018**
(21) Application number: 17275196.8
(22) Date of filing: 22.12.2017
(51) Int. Cl.: A61F 2/07, A61F 2/06

(54) **ARTICULAR VASCULAR IMPLANTS USING POLYMAGNETS**

(30) Priority: 29.12.2016 US 201615394228
(71) Applicant: Cook Medical Technologies LLC, Bloomington, IN 47404 (US)
(72) Inventor: Skender, Davorin Kevin, Bloomington, IN 47403 (US); Spindler, Ralf, Bloomington, IN 47401 (US)
(74) Representative: Williams Powell

(57) **Abstract**

An implantable medical device includes a first section and a second section, and an interface region between the first and second sections. A magnet array (40, 42, 44, 46) overlies each of opposing surfaces of each of the first and second sections at least at the interface region. The magnet arrays (40, 42, 44, 46) include a plurality of magnetic elements arranged in a predetermined pattern, each of the plurality of magnetic elements having a north and a south pole. The predetermined pattern is such that the north and south poles of the magnetic elements in each magnetic arrays are conjugated with one another so as to define a variable magnetic field between the first and second sections.

## Description

### Technical Field text

The disclosure relates to implantable medical devices, such as segmented stent grafts having bifurcated segments and fenestration assemblies, and the like, and, more particularly, to implantable medical devices that include magnet arrays configured to manipulate the articulation of the stent segments.

### Background

Implantable medical devices are known in many forms and for treating many medical conditions. Examples include stents, grafts, filters, occluders, valve replacement prostheses and so on. Such devices are generally introduced into the patient endoluminally through a remote percutaneous entry point. In order to achieve this, the medical device is loaded onto a carrier at a distal end of an introducer assembly with the device being held in a radially compressed configuration. The introducer assembly is fed into the patient's vasculature from the percutaneous entry point until its distal end is located at the treatment site. Once so positioned, the medical device is released from the carrier and expanded until the device engages the vessel wall to be held thereby. The device can be of a type which expands automatically, achieved by use of a spring material, shape memory material and so on. Other types of device are plastically deformable and expanded by a separate mechanism, for instance by inflation of a delivery balloon on which the device is held in crimped form.

Stent grafts are used for treatment of vasculature in the human or animal body to bypass a repair or defect in the vasculature. For instance, a stent graft may be used to span an abdominal aortic aneurism. In many cases, however, such damaged or defected portion of the vasculature may include a branch vessel, such as a mesenteric artery or a renal artery. Bypassing such an artery without providing blood flow into the branch artery can cause problems and hence fenestration assemblies are provide in the wall of a stent graft which, when the stent graft is deployed, is positioned over the opening to the branch vessel. Another stent graft can be deployed through the fenestration into the branch vessel to provide a blood flow path to the branch artery.

Typically the stent grafts must be custom made to conform to the unique branching vasculature of the patient. Custom made devices require a long lead time and extreme precision in the design and fabrication of the device. Further, such custom manufacturing requires considerable to time from the first stages of device manufacturer to delivery to the physician/patient.

### BRIEF SUMMARY

Aspects of the present invention seek to provide an improved medical device.

According to an aspect of the invention, there is provided an implantable medical device as in claim 1.

The variable magnetic field may be a variable magnetic field strength.

According to an aspect of the invention, there is provided an implantable medical device comprising:
a first section and a second section;
an interface region between the first and second sections;
a magnet array overlying each of opposing surfaces of each of the first and second sections at least at the interface region, the magnet arrays including a plurality of magnetic elements arranged in a predetermined pattern, each of the plurality of magnetic elements having a north and a south pole,
wherein the predetermined pattern is such that the north and south poles of the magnetic elements in each magnetic arrays are conjugated with one another so as to define a variable magnetic field strength between the first and second sections.

The present disclosure can provide an implantable medical device that incorporates magnetic elements, such that the device has the ability to be readily adapted a patient's anatomic configuration.

Preferred embodiments can provide an implantable medical device in which the configuration of the device can be customized to a particular patient more rapidly than is now allowed by current methods.

According to an aspect of the invention there is provided a stent graft comprising: a first section and a second section, the second section having a preferred rotational position with respect to the first section; an interface region between the first and second sections; a magnet array overlying each of opposing surfaces of each of the first and second sections at least at the interface region, the magnet arrays including a plurality of magnetic elements arranged in a predetermined pattern, each of the plurality of magnetic elements having a north and a south pole, wherein the predetermined pattern is such that the north and south poles of the magnetic elements in each of the magnetic arrays are conjugated with one another so as to define a variable magnetic field between the first and second sections, wherein the magnetic field is such that the second section assumes the preferred rotational position with respect to the first section upon contact of the first and second sections.

In accordance with an embodiment of the disclosed subject matter, an implantable medical device includes a first section and a second section, and an interface region between the first and second sections. A magnet array overlies each of opposing surfaces of each of the first and second sections at least at the interface region. The magnet arrays include a plurality of magnetic elements arranged in a predetermined pattern, each of the plurality of magnetic elements having a north and a south pole. The predetermined pattern is such that the north and south poles of the magnetic elements in each magnetic arrays are conjugated with one another so as to define a variable magnetic field between the first and second sections.

The magnet arrays at the interface region may each comprise a regular array of magnetic elements.

The magnet arrays at the interface region may each comprise an irregular array of magnetic elements.

The magnetic arrays may include extensions that provide a rotational detent with respect to relative rotation of the first and second sections.

In accordance with another embodiment of the disclosed subject matter, a stent graft includes a first section and a second section. The second section has a preferred rotational position with respect to the first section. An interface region resides between the first and second sections. A magnet array overlies each of opposing surfaces of each of the first and second sections at least at the interface region. The magnet arrays include a plurality of magnetic elements arranged in a predetermined pattern, each of the plurality of magnetic elements having a north and a south pole. The predetermined pattern is such that the north and south poles of the magnetic elements in each of the magnetic arrays are conjugated with one another so as to define a variable magnetic field between the first and second sections. The magnetic field is such that the second section assumes the preferred rotational position with respect to the first section upon contact of the first and second sections.

### BRIEF DESCRIPTION OF THE DRAWINGS

Embodiments of the invention are described below, by way of example only, with reference to the accompanying drawings.
FIG. 1 illustrates a exploded perspective view of a composite stent graft in accordance with an embodiment of the disclosed subject matter;
FIG. 2 illustrates an assembled perspective view of the composite stent graft of FIG. 1;
FIG. 3A illustrates magnetic element arrays and a schematic view of stent graft sections assembled in accordance with an embodiment of the disclosed subject matter and FIG. 3B illustrates a schematic view of stent graft sections having an angled circumferential interface;
FIGs. 4A and 4B illustrate regular magnet arrays in accordance with an embodiment of the disclosed subject matter;
FIG. 5 illustrates a perspective view a fenestration assembly in a stent graft segment;
FIG. 6 illustrates a cross-sectional view of the stent graft segment of FIG. 5;
FIG. 7 illustrates a partial sectional view of a stent graft in accordance with an embodiment of the disclosed subject matter deployed in a human aorta;
FIG. 8 illustrates a schematic view of stent graft sections of a fenestration assembly in accordance with an embodiment of the disclosed subject matter;
FIGs. 9A and 9B illustrate magnet arrays configured to assemble stent graft sections in accordance with an embodiment of the disclosed subject matter;
FIG. 10A and 10B illustrate magnet arrays configured to assemble stent graft sections in accordance with another embodiment of the disclosed subject matter;
FIGs. 11A, illustrates an exemplary embodiment of a magnet array, FIG. 11B illustrates an embodiment of a sectional view of the magnet array of FIG. 11A, and FIG. 11C is a plot showing an exemplary magnetic field contour of the magnet array of FIG. 11A; and
FIG. 12A, illustrates a wire bonded to a polymer film or graft material, and FIG. 12B illustrates an embodiment of a wire sutured to a graft material.

### DETAILED DESCRIPTION OF PREFERRED EMBODIMENTS

Embodiments described herein provide an implantable medical device comprising a first section and a second section; an interface region between the first and second sections; and a magnet array overlying each of opposing surfaces of each of the first and second sections at least at the interface region, the magnet arrays including a plurality of magnetic elements arranged in a predetermined pattern, each of the plurality of magnetic elements having a north and a south pole, the predetermined pattern being such that the north and south poles of the magnetic elements in each magnetic arrays are conjugated with one another so as to define a variable magnetic field strength between the first and second sections.

In embodiments, as described herein, the magnetic field has a spatial variation in magnetic strength, such that, when assembled, the first and second sections can for example assume a specific orientation relative to each other. As described below, in some embodiments the individual magnets can be programmed to have different magnetic strength.

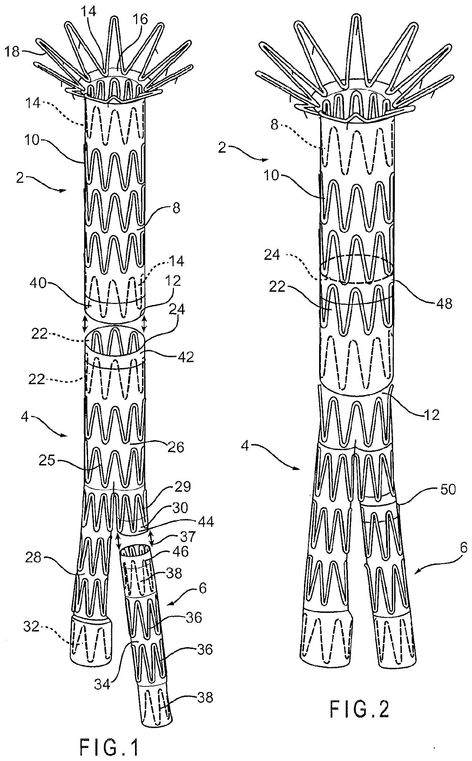

FIGS. 1 and 2, illustrate an exemplary composite stent graft that includes a first or proximal graft portion 2, a second or distal graft portion 4 and a leg graft portion 6. The first graft portion 2 comprises a fabric material graft body 8 of substantially tubular form with self-expanding zig zag stents 10 on the outside along most of its length and self-expanding zig zag stent section 14 within the tubular body 8 at the proximal end 16 and distal end 12. Extending from the proximal end 16 is a supra-renal zig zag stent 18 with barbs 20 extending distally to provide fixation into the wall of the aorta.

The zig-zag stents are also well known as Gianturco Z-stents commercially available from William A Cook Australia Pty Ltd, Brisbane, Australia or Cook Inc, Bloomington, Ind., USA. The graft material is typically DACRON™ material available from a number of medical graft manufacturers.

The zig zag stent within the proximal end 16 of the first graft portion 2 assists with sealing of the graft against the walls of the aorta and the external zig zag stents provide a smooth inner surface for the flow of blood through the graft. The internal zig zag stent section 14 at the distal end section 12 provides an outer surface of the tubular body 8, which is smooth and can seal within the proximal end of the second graft portion 4 when it is deployed within the second graft portion 4.

The second graft portion 4 comprises a fabric material graft body 26 and has an internal zig zag stent 22 at its proximal end 24 so that the outer surface of its tubular body 26 is smooth and can seal within the distal end of the first graft portion 2 when it is deployed within the first graft portion 2. The external zig zag stents 25 provide a smooth inner surface for the flow of blood through the graft. The second graft portion 4 is bifurcated and has a leg graft portion 6.

Towards the distal end of the second graft portion 4 the tubular body 26 bifurcates into a longer leg section 28 and a shorter leg 30 each of which has a zig zag stent section 29 on its outside surface except the terminal zig zag stent section 32 on the longer leg.

The leg graft portion 6 which is adapted to extend into the contralateral-iliac artery is comprised from a tubular fabric material body 34 with outside zig zag stents 36 along its length except for internal zig zag stents 38 at its proximal and distal ends. Those skilled in the art will appreciate that the bifurcated graft segment has a preferred orientation with respect to the first segment, depending upon the particular tortuosity of the patient's vasculature.

As will subsequently be described in more detail, in one embodiment, magnet arrays 40 and 42 overlie opposing surfaces at the distal end 12 of the first graft portion 2 and at the proximal end 24 of the distal graft portion 4. Further, magnet arrays 44 and 46 overlie opposing surfaces at the distal end the shorter leg 30 and at the proximal end 37 of leg graft portion 6.

FIG. 2 illustrates the assembled graft in a configuration in which the connecting end 24 of the second graft portion 4 is deployed within the distal connecting end 12 of the first graft portion 2. It will be realized that the amount of overlap between the first graft portion 2 and the second graft portion 4 can be varied for different lengths of an aorta from the renal arteries to the aortic bifurcation. It is preferable, however, that there is at least a longitudinal or axial overlap of two stents. This means that there will be a smooth inner surface of one portion engaged against a smooth outer surface of the other portion at an interface region 48. Similarly, the leg graft portion 6 is deployed with its proximal end 37 within the short leg 30 of the second graft portion 4 at an interface region 50.

Those skilled in the art will appreciate that, in addition to the examples illustrated in the composite stent graft of FIGs. 1 and 2, the disclosed magnet arrays can be included in any or all of the sealing interfaces where two graft sections engage in an articulating joint. For example, magnet arrays can be included in the sealing interface of the terminal zig zag stent section 32 and the longer leg section 28. Further, the disclosed magnet arrays can be included in the articulating interface of zig zag stent section 36 and zig zag stent section 38. Moreover, magnet array can also be included at the proximal end of zig zag stent section 14 in an articulating interface. Accordingly, the magnet arrays disclosed herein can be widely applied to articulating interfaces of graft sections as needed to accommodate the tortuosity of a patient's vasculature.

The graft in FIG. 2 is assembled in what is also known as a top down approach or assembly. The physician will deploy the proximal graft portion 2 first in the aorta of a patient followed by deploying or placing the distal graft portion 4 in the aorta with the proximal end 24 of the distal graft portion 4 inside the distal end 12 of the proximal graft portion 2. In accordance with an embodiment, relative rotation of the first and second stent portions 2 and 4, and the leg graft portion 6 and the short leg 30 can be determined by the particular pattern of magnetic elements in the corresponding magnet arrays.

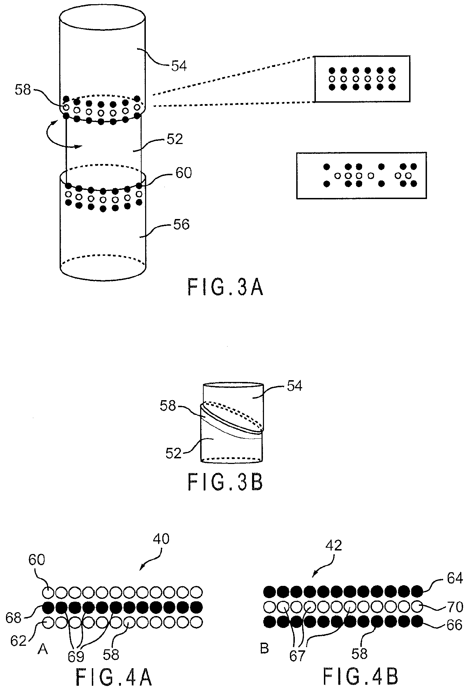

The principal of operation of the magnet arrays will now be described in connection with schematic representation of graft segments. FIG. 3A illustrates, a schematic view of a first graft section 52 inserted into end portions of a second graft section 54 and a third graft section 56. At an interface region 58 resides between end portions of first and second graft sections 52 and 54. Further, an interface region 60 resides between first and third graft sections 52 and 56. In each interface region, a magnet array overlies opposing surfaces of the graft sections in the annular space between the end portions of the graft sections. As depicted in FIG. 3A, the magnet arrays can be configured in a regular array or an irregular array depending upon the particular application for a segmented graft.

FIG. 3B illustrates another embodiment of first graft section 52 inserted into the end portion of second graft section 54 at an angled circumferential interface. In FIG. 3B, rather than being particular to the longitudinal axis of sections 52 and 54, interface region 58 not perpendicular thereto, but instead is angled or off-set with respect to the longitudinal axis of sections 52 and 54. Such an off-set angle is applicable to branching graft sections, such as those described above. The magnet arrays are circumferentially positioned in angled articulating interface region 58 in a similar manner as shown in FIG. 3A.

FIGs. 4A and 4B illustrate an exemplary embodiment of magnet arrays 40 and 42. Each array includes a plurality of magnetic elements 58 arranged in regular row and columns. In the exemplary embodiment, magnetic elements having opposite polarity are arranged in a conjugated pattern, such that when magnet arrays 40 and 42 are oppositely positioned, magnetic elements having opposite polarity will align with each other. For example, the magnetic elements in first and second rows 60 and 62 in magnet arrays 40 have an opposite polarity as the magnetic elements in first row 64 and second row 66 of magnet array 42. The magnetic elements in the middle rows 68 and 70 can also have opposite polarity to one another.

The arrangement described above is only one of number of different polarity patterns that can be employed depending upon the particular application. Also, the magnetic elements can be arranged such that the segments will align with each other in a preferred orientation. For example, the number of magnetic elements having opposing polarity in each of the rows in magnet arrays 40 and 42 can be varied, such that when graft portions 2 and 4 are deployed by placing distal graft portion 4 in the aorta with proximal end 24 of the distal graft portion 4 inside distal end 12 of graft portion 2, graft portion 4 can assume a preferred rotational orientation with respect to graft portion 2. Accordingly, the magnetic field strength between the magnet arrays can be determined by the particular arrangement of the relative position of the magnetic elements in the magnet arrays.

Further, in accordance with another embodiment, the magnetic strength of the magnetic elements can vary between one another. By varying the relative magnetic strength among the magnetic elements, the strength of the magnetic field between the magnet arrays can be specifically designed to have a particular contour, such that a controlled degree of flexibility between the segments of the stent graft is attained. Those skilled in the art will appreciate that a variation in the magnetic field strength can provide stress compensation between the segments of the stent graft that will accommodate the natural motion of the patient's body and any on-going geometric changes that increase vessel tortuosity.

In yet another embodiment, the arrays illustrated in FIGs. 4A and 4B can be irregular arrays in which certain of positions in the array are not occupied by magnetic elements. For example, selected positions 67 and 69 can be open spaces in the magnet array, thus providing an irregular array of magnetic elements. An irregular array can also be provided by including a larger number of magnetic elements in one of the rows versus the number in other rows.

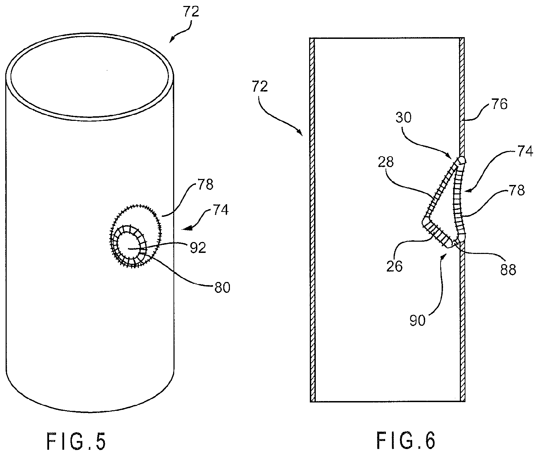

FIGs. 5 and 6 illustrate a fenestrated stent graft that includes a tubular body 72 and may include self-expanding or balloon expandable stents in a well-known manner but these are not shown in these illustrations. A fenestration assembly 74 is in a wall 76 of the tubular body 72. The fenestration assembly 74 includes an outer ring 78 and an inner ring 80. Joining the inner and outer rings is a substantially funnel or frusto-conical shaped portion of graft material 82 which is skewed so that the inner ring 80 is much closer to the outer ring 78 at the lower end 84 of the fenestration assembly than the upper end 86. The short piece of material 88 at the end 90 in effect provides a hinge arrangement between the outer ring 78 and the inner ring 80. The inner ring 80 provides an aperture or fenestration 92 through which a side branch stent graft may be deployed.

The inner ring 80 can move angularly with respect to the outer ring 78 so as to allow for misalignment of the fenestration with the branch vessel when the stent graft 72 is positioned within a body lumen.

It will be noted, that the open aperture in the smaller ring is directed towards one end of the stent graft. This can be varied to face towards one end or the other of the stent graft depending upon what direction the physician is intending to approach the fenestration. For instance in deployment into the aorta a physician may use either a brachial or a femoral approach.

Those skilled in the art will recognize that the fenestrated stent graft illustrated in FIGs. 5 and 6 is very simplified schematic depiction of such stent grafts. In accordance with the disclosed subject matter a number of stent grafts can benefit from the magnet arrays disclosed herein, such as an iliac branch device (IBD), T-Branch, and A-Branch devices can include magnetic moveable branches as disclosed herein. With respect to the IBD, the current designs include an external branch that is predominantly rigid and not conforming to the anatomy and accurate placement is necessary. Whereas, with an IBD incorporating a magnetically attached branch provides added flexibility due to magnet arrays. Advantageously, surgeons may not need to achieve perfectly accurate placement using one of the inventive devices, thus allowing surgeons to rapidly place an IBD device in the vasculature of a patient.

Those skilled in the art will recognize that numerous different vascular branching structures exist. For example, T-branch stent grafts are known in the art with up to four external branches in a downward (caudal) pointing direction. A stent graft incorporating the disclosed magnet arrays can better angulate to accommodate challenging anatomies and widen the scope of use (inclusion/IFU criteria). The magnetically connected branches being more flexible to optimize the alignment with the branch vessel and allow a larger alignment margin for placement of the device. Further, migration or movement of the T-branch stent graft would not immediately compromise the branches either due to the ability of the magnetically attached branches to accommodate movement and continue to facilitate optimal flow to the branch vessels.

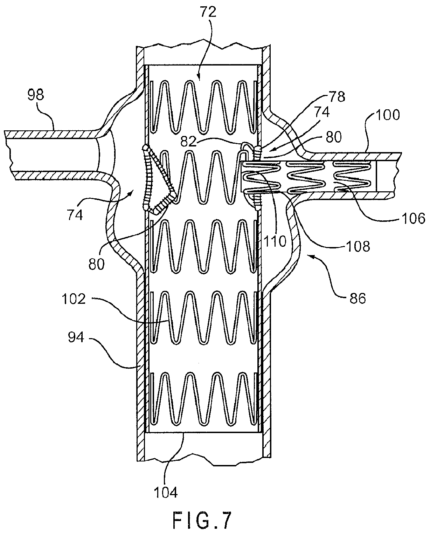

FIG. 7 illustrates a schematic diagram of an aorta 60 which has an aneurysm generally shown as 96. The aneurysm or expanded portion of the aorta in this case includes the entrances to the renal arteries 98 and 100 within the aneurysmal region. A stent graft generally shown as the tubular body 72 has been deployed to bridge or span the aneurism 96. It will be noted that in the stent graft 72 there are two fenestration assemblies 74 of the type shown in FIGs. 5 and 6 and these have been positioned so that their apertures approximate the positions of the renal arteries 98 and 100. The stent graft 72 includes a number of self-expanding zig zag or Z stents 102 of the well-known Gianturco type.

In the left hand side of FIG. 7 the stent graft is shown in an as deployed condition before a side branch stent graft has been deployed and on the right hand side of the drawing in FIG. 7, a side branch stent graft has been deployed through the fenestration.

As can be seen on the left hand side of the drawing, the fenestration assembly has its inner ring 80 and hence its opening facing slightly towards the distal end 104 of the stent graft so that when a physician is attempting to deploy a guide wire from the aorta into the side branch through the stent graft 72 it will be somewhat easier to guide the guide wire through the aperture in the fenestration assembly 74.

A bridging stent graft 106 is deployed in the right hand fenestration assembly 74. After the bridging stent graft 106 has been deployed, the inner ring 80 of the fenestration assembly is engaged around the outside of the bridging stent graft 106 and the inner ring 80 has hinged to a more vertical position which means that the graft material 82 joining the inner ring 80 and the outer ring 76 of the fenestration assembly 74 is now not taut but in this condition still provides a leak proof seal for the fenestration.

The resilient inner ring provides a good sealing and retention surface for the proximal end of the bridging stent graft 106. The bridging stent graft 106 may have stents of a balloon expandable or of a self-expanding type. The bridging stent graft 106 includes a number of self-expanding zig zag or Z stents 108 of the well-known Gianturco type.

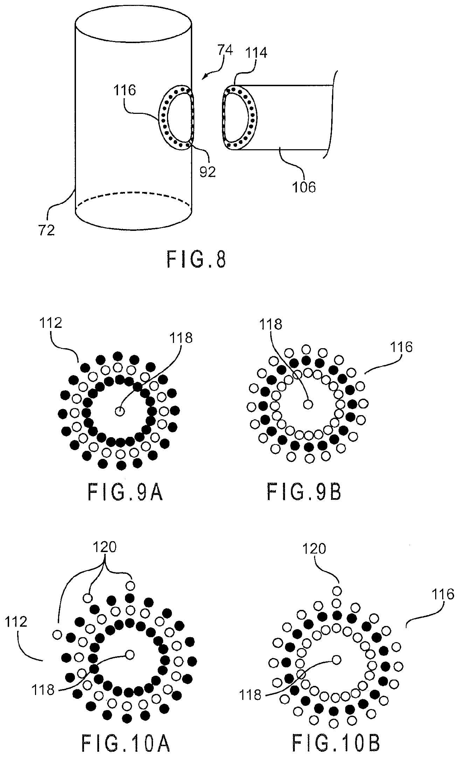

In accordance with an exemplary embodiment, arrays of magnetic elements are included in an interface region 110 between abutting portions of stent graft 72 and bridging stent graft 106. A magnet arrays overlie both the periphery of fenestration 92 and the circumferential end of bridging stent graft 106 in interface region 110. A schematic diagram of fenestration assembly 74 is illustrated in FIG. 8.

A magnet array 112 overlies an end surface 114 of bridging stent graft 106. A magnet array 116 overlies the periphery of fenestration 92 in stent graft 72. The magnetic elements in each array are conjugated so as to provide a magnetic field at the interface region 110. An exemplary embodiment of magnet arrays 112 and 116 is illustrated in FIGs. 9A and 9B. The magnetic elements in the arrays are arranged such that rotation is permitted about a central axis 118 of fenestration 92 and articulation of bridging stent graft 106 is enabled. As such, prior to deployment, the physician can position bridging stent graft 106 in a variety of positions relative to stent graft 72 to correspond with the particular anatomic configuration of the patient's vasculature. Further, the magnetic strength of individual ones of the magnetic elements in each array can be varied such that a preferred rotational orientation is obtained for one segment relative to the other segment.

While the p-branch devices such as that schematically illustrated in FIG. 8 are widely used, there are patients who are not candidates a p-branch device. The circumferential rotation of fenestration stent grafts, such as that schematically illustrated in FIG. 8, expends the utility of the device because one device design may be able to treat a large number of patients due to the ability of the fenestration stent graft having magnet arrays to rotate around the circumference of the device.

Another embodiment of the magnet arrays of FIGs. 9A and 9B is schematically illustrated in FIGs. 10A and 10B. In each array, one or more extensions 120 protrude from the outermost ring of magnetic elements. In accordance with the illustrated embodiment, a preferred rotational orientation about central axis 118 is obtained by the relative magnitude of the magnetic attraction between magnet arrays 112 and 116. The extensions 120 form a type of detent, such that the relative degree of rotation of one segment with respect to the other segment can be predetermined. In some embodiments, the magnetic field strength can vary among the extensions to provide a ratchet effect as the physician rotates the segments relative to one another, prior to deployment. Accordingly, a preferred orientation of the segments can be obtained and locked into position if desired, prior to deployment.

The arrays of magnetic elements can be incorporated into the walls of the stent graft segments in a number of different configurations. For example, the magnetic elements can be incorporated into a polymer cover material. A number of different biocomaptible graft materials can be used to cover the stents. Examples of graft materials include polyesters, such as Dacron™ (polyethylene terphthalate or PET), fluorinated polymers, such as PTFE (polytetrafluoroethylene) and Teflon™(expanded polytetrafluoroethylene or ePTFE); polyurethanes such as THORALON™; polyamides such as nylon; or any other suitable material such as coliagenous extracellular matrix (ECM) material including small intestine submucosa (SIS), which is commercially available from Cook Biotech, West Lafayette, Ind., U.S.A. Besides SIS, examples of ECM's include pericardium, stomach submucosa, liver basement membrane, urinary bladder submucosa, tissue mucosa, and dura mater.

Graft materials may include sheets containing a biocompatible polymer. Examples of biocompatible polymers from which sheets can be formed include polyesters, such as polyethylene terephthalate, polylactide, polyglycolide and copolymers thereof; fluorinated polymers, such as polytetrafluoroethylene (PTFE), expanded PTFE and poly(vinylidene fluoride); polysiloxanes, including polydimethyl siloxane; and polyurethanes, including polyetherurethanes, polyurethane ureas, polyetherurethane ureas, polyurethanes containing carbonate linkages and polyurethanes containing siloxane segments. In addition, materials that are not inherently biocompatible maybe subjected to surface modifications in order to render the materials biocompatible. The graft material may include a biocompatible polyurethane.

A variety of other biocompatible polyurethanes/polycarbamates and urea linkages (hereinafter "CON type polymers") may also be employed. These include CON type polymers that preferably include a soft segment and a hard segment. The segments can be combined as copolymers or as blends. For example, CON type polymers with soft segments such as PTMO, polyethylene oxide, polypropylene oxide, polycarbonate, polyolefin, polysiloxane (i.e. polydimethylsiloxane), and other polyether soft segments made from higher homologous series of diols may be used. Mixtures of any of the soft segments may also be used. The soft segments also may have either alcohol end groups or amine end groups. The molecular weight of the soft segments may vary from about 500 to about 5,000 g/mole.

Other applicable biocompatible polyurethanes include those using a polyol as a component of the hard segment. Polyols may be aliphatic, aromatic, cycloaliphatic or may contain a mixture of aliphatic and aromatic moieties. For example, the polyol may be ethylene glycol, diethylene glycol, triethylene glycol, 1,4-butanediol, 1,6-hexanediol, 1,8-octanediol, propylene glycols, 2,3-butylene glycol, dipropylene glycol, dibutylene glycol, glycerol, or mixtures thereof. Biocompatible CON type polymers modified with cationic, anionic and aliphatic side chains may also be used. See, for example, U.S. Pat. No. 5,017,664. Other biocompatible CON type polymers include: segmented polyurethanes, such as BIOSPAN; polycarbonate urethanes, such as BIONATE; and polyetherurethanes, such as ELASTHANE; (all available from POLYMER TECHNOLOGY GROUP, Berkeley, Calif.). Other biocompatible CON type polymers can include polyurethanes having siloxane segments, also referred to as a siloxane-polyurethane.

Graft materials also may be woven (including knitted) textiles or nonwoven textiles. Nonwoven textiles are fibrous webs that are held together through bonding of the individual fibers or filaments. The bonding can be accomplished through thermal or chemical treatments or through mechanically entangling the fibers or filaments. Because nonwovens are not subjected to weaving or knitting, the fibers can be used in a crude form without being converted into a yarn structure. Woven textiles are fibrous webs that have been formed by knitting or weaving. The woven textile structure may be any kind of weave including, for example, a plain weave, a herringbone weave, a satin weave, or a basket weave. A textile material contains fibers and interstices between the fibers.

The graft material may be a reconstituted or naturally-derived collagenous material. Such materials that are at least bioresorbable will provide advantage in embodiments of the present invention, with materials that are bioremodelable and promote cellular invasion and ingrowth providing particular advantage. Suitable bioremodelable materials can be provided by collagenous extracellular matrix materials (ECMs) possessing biotropic properties, including in certain forms angiogenic collagenous extracellular matrix materials. For example, suitable collagenous materials include ECMs such as submucosa, renal capsule membrane, dermal collagen, dura mater, pericardium, fascia lata, serosa, peritoneum or basement membrane layers, including liver basement membrane. Suitable submucosa materials for these purposes include, for instance, intestinal submucosa, including small intestinal submucosa, stomach submucosa, urinary bladder submucosa, and uterine submucosa.

Preferably, the magnetic elements are laminated into, bonded with, or attached to a polymer material or a graft material. In one example, a magnetic pattern is printed on a ferromagnetic material. Methods for producing an array of magnetic elements or a wire including the magnetic elements are known in the art. See for example, U.S. Pat. No. 7,800,471, which is incorporated by reference herein. The polarity of the magnets, the magnetic strength, and the pattern of magnets in the ferromagnetic material can be computer programmed to produce a desired pattern in the ferromagnetic material. The ferromagnetic material can then be assembled with one of more of the graft materials described above. Further, the ferromagnetic material can be formed as a wire and the wire integrated with or attached to the graft material.

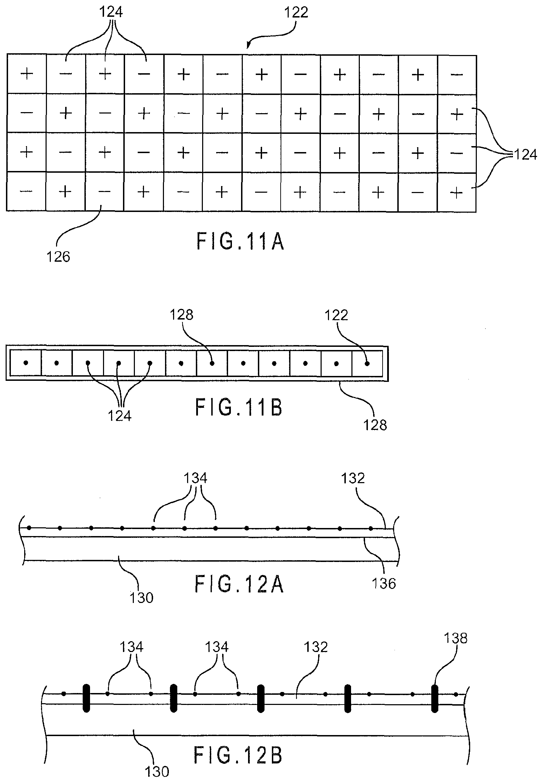

FIG. 11A illustrates a magnet array 122 having a plurality of magnetic elements 124. The magnetic elements 124 are arranged in a regular array with alternating positive and negative poles. The magnetic elements are printed in a sheet of ferromagnetic material 126 in accordance with techniques known in the art, as described above. In one embodiment, the ferromagnetic material can be Iron (Fe), Cobalt (Co), or Nickel (Ni), and alloys thereof. The magnetic elements 124 can be arranged differently depending upon the particular application of the magnet array in a stent graft, as described in the foregoing embodiments. Further, the magnetic strength of individual ones of the plurality of magnetic elements can vary based on a desired magnetic field strength profile.

In accordance with the disclosed subject matter the magnet arrays can be attached to stent graft segments in a variety of attachment configurations. For example, the magnet array can be laminated between layers of a polymer film. In other embodiments, the magnet arrays can be directly bonded to the graft material. In yet another configuration, the magnet arrays can be fabricated in the form of ferromagnetic wires and bonded or sutured to the graft material. Still further, the magnet arrays can be contained within material pouches, formed a polymer film or from grant material, and bonded or sutured to the stent graft segments.

FIG. 11B illustrates a sectional view ferromagnetic material 126 and magnetic elements 124 laminated between two sheets of a polymer film 128. In accordance with the embodiment, the magnet array 122 is mounted in a stent graft section, as described above. The sheets of polymer film 128 can be bonded to the graft material of a stent graft as described above. In other embodiments, the sheets of polymer film 128 can be sections of a pouch that is bonded or sutured to the graft material covering a stent graft. In yet another embodiment, the polymer film is replaced by graft material.

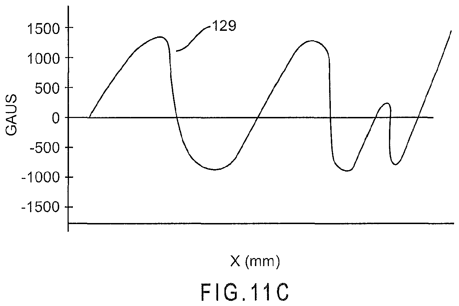

FIG. 11C is a representative plot of magnetic field strength versus distance across the magnet array 122. It provides an exemplary graphical representation of a spatial variation in magnetic field strength. The magnetic field contour 129 is a result of the variation in the individual magnetic elements 124 in the magnet array. In accordance with an embodiment of the disclosed subject matter, the magnetic elements can be printed so as to have different magnetic strength relative to one another. In this way, the magnetic field is programmed to have a particular contour so as to achieve a preferred arrangement the stent segments relative to one another, as described above.

FIG. 12A is a sectional view of a graft material130 having a wire 132 of ferromagnetic material bonded thereto. Magnetic elements 134 are printed along the wire 132. The wire is bonded to the graft material by a layer of adhesive material 136. The wire can be positioned around the perimeters of a stent graft segment, or arranged in various patterns on the surface of the graft material.

FIG. 12B is a sectional view of wire 132 sutured to graft material 130 by sutures 138. The wire includes a plurality of the magnetic elements 134 positioned along the wire at a regular or irregular interval.

By providing graft segments having magnet arrays or wires containing magnetic elements, as described above, standardized stent graft segments can be supplied and the particular geometric aspects of the articulated stent segments determined by specifically programmed magnet arrays that are appropriately positioned in the stent segments. The arrays are programmed to cause the graft segments to articulate in such a way as to correspond to the vascular tortuosity of a particular patient. This technique reduces the manufacturing time to produce a graft section, as compared with the time required to manufacture customized graft segments.

Those skilled in the art will recognize that the magnet arrays and graft segments schematically illustrated in FIGs. 3 and 8 can be combined into a wide variety of segmented stent graft designs. For example, U.S. Pat. No 6,524,335 entitled "Endoluminal Aortic Stents" discloses a fenestrated prosthesis for placement where there are intersecting arteries. This feature and other features disclosed in U.S. Pat. No. 6,524,335 could be fitted with a segmented stent graft as disclosed herein, the disclosure of is herewith incorporated by reference herein. U.S. Pat, No. 6,974,471 entitled "Prosthesis For Curved Lumens" discloses prostheses with arrangements for bending the prosthesis for placement into curved lumens. This feature and other features disclosed in U.S. Pat, No. 6,974,471 could be fitted with a segmented graft as disclosed herein, and is hereby incorporated by reference herein. U.S. Pat. No. 7,998,187 entitled "Stent Graft Connection Arrangement" discloses a fenestrated stent grafts having connection sockets for placement where there are intersecting arteries. This feature and other features disclosed in U.S. Pat. No. 7,998,187 could be fitted with a segmented graft as disclosed herein, and is hereby incorporated by reference herein. U.S. Pat. No. 9,072,621 entitled "Fenestrated Stent Grafts" discloses a fenestrated stent grafts having connection sockets for placement where there are intersecting arteries. This feature and other features disclosed in U.S. Pat. No. 9,072,621 could be fitted with a segmented graft as disclosed herein, and is hereby incorporated by reference herein.

Thus, is apparent that there has been described an implantable medical device including magnetic elements that fully provides the advantages set forth above. Those skilled in the art will recognize that numerous modifications and variations can be made without departing from the scope of the invention. For example, the magnet arrays can be arranged in a stent graft so as to hold open the stent segment, or to unfold a stent segment before or during delivery. Accordingly, all such variations and modifications are within the scope of the appended claims and equivalents thereof.

All optional and preferred features and modifications of the described embodiments and dependent claims are usable in all aspects of the invention taught herein. Furthermore, the individual features of the dependent claims, as well as all optional and preferred features and modifications of the described embodiments are combinable and interchangeable with one another.

The disclosures in United States patent application number 15/394,228, from which this application claims priority, and in the abstract accompanying this application are incorporated herein by reference.

## Claims

1. An implantable medical device comprising:
a first section and a second section;
an interface region between the first and second sections;
a magnet array overlying each of opposing surfaces of each of the first and second sections at least at the interface region, the magnet arrays including a plurality of magnetic elements arranged in a predetermined pattern, each of the plurality of magnetic elements having a north and a south pole,
wherein the predetermined pattern is such that the north and south poles of the magnetic elements in each magnetic arrays are conjugated with one another so as to define a variable magnetic field between the first and second sections.

2. The implantable medical device of claim 1, wherein the first and second sections each comprise a tubular structure having different diameters, such that the first section has a smaller diameter than the second section and the interface region comprises an annulus between end regions of the first and second sections.

3. The implantable medical device of any preceding claim, wherein the first and second sections comprise articulating segments of a stent graft, and the variable magnetic field of the magnet array at the interface region is such that the first and second sections have a preferred rotational orientation with respect to each other.

4. The implantable medical device of any preceding claim wherein the predetermined pattern comprises a circumferential region at the interface region.

5. The implantable medical device of any preceding claim, wherein the predetermined pattern comprises a linear region at the interface region and oriented parallel to a longitudinal axis of the first and second sections.

6. The implantable medical device of any preceding claim, wherein the first section comprises a stent graft having a fenestration and the second section comprises a bridging graft and the interface region comprises a periphery of the fenestration.

7. The implantable medical device of any preceding claim, wherein each of the magnet arrays at the interface region comprise a regular array of magnetic elements.

8. The implantable medical device of any preceding claim, wherein each of the magnet arrays at interface region comprise an irregular array of magnetic elements.

9. The implantable medical device of any preceding claim, wherein the second section comprises an articulating segment of a bridge graft, and a variable magnetic field of the magnet arrays at the interface region is such that the second section has a preferred rotational orientation with respect to the first section.

10. The implantable medical device of any preceding claim, wherein the magnet arrays include extensions that provide a rotational detent with respect to relative rotation of the second section with respect to the first section, wherein the medical device is optionally a stent graft.

11. The implantable medical device of any preceding claim, wherein the magnet arrays each comprise the plurality of magnetic elements disposed in a flexible ferromagnetic material.

12. The implantable medical device of claim 11, wherein the flexible ferromagnetic material comprises:
a wire attached to a surface of each of the first and second segments; and/or
a polymer film attached to a surface of each of the first and second segments at least at the interface.

13. The implantable medical device of claim 11 or 12, wherein the flexible ferromagnetic material resides between layers of a graft fabric.

14. The implantable medical device of any preceding claim, wherein a magnetic strength of each of the plurality of magnetic elements varies with respect one another.

15. The implantable medical device of any preceding claim, wherein:
the device is a stent graft;
the second section has a preferred rotational position with respect to the first section;
the magnetic field is such that the second section assumes the preferred rotational position with respect to the first section upon contact of the first and second sections;
the second section optionally comprises a bifurcated graft segment.
